# EUROPEAN PATENT APPLICATION

(11) **EP 2 138 173 A1**
(43) Date of publication of application: **30.12.2009**
(21) Application number: 08011634.6
(22) Date of filing: 26.06.2008
(51) Int. Cl.: A61K 31/13, A61K 31/724, A61P 25/00, A61P 25/28, A61P 25/16, A61P 25/04, A61P 25/08, A61P 29/00, A61P 9/10, A61P 25/24

(54) **Pharmaceutical compositions comprising aminoadamantane derivatives**

(71) Applicant: Merz Pharma GmbH & Co. KGaA, 60318 Frankfurt (DE)
(72) Inventor: Plitt, Kirsten, 68161 Mannheim (DE); Purmann, Brigitte, 63165 Mühlheim (DE); Szlak-Freier, Alda, 60431 Frankfurt/Main (DE); Hauptmeier, Bernhard, 63571 Gelnhausen (DE)
(74) Representative: Ricker, Mathias

(57) **Abstract**

The present invention relates to pharmaceutical compositions comprising 1-aminoadamantane derivatives and a cyclodextrin, which compositions exhibit advantageous safety, convenience, and dosing characteristics. The compositions of the instant invention find particular application in the treatment of various diseases and conditions of the CNS, including those involving the impairment of cognitive function or dementia, such as Alzheimer's disease.

## Description

### FIELD OF THE INVENTION

The present invention relates to pharmaceutical compositions comprising 1-aminoadamantane derivatives and a cyclodextrin, which compositions exhibit advantageous safety, convenience, and dosing characteristics. The compositions of the instant invention find particular application in the treatment of various diseases and conditions of the CNS, including those involving the impairment of cognitive function or dementia, such as Alzheimer's disease or for the treatment of behavioural disorders such as autistic spectrum disorders.

### BACKGROUND OF THE INVENTION

Dementia is commonly defined as a chronic deterioration of intellectual function and other cognitive skills severe enough to interfere with the ability to perform activities of daily living. Alzheimer's disease is a form of dementia which is characterized by "...progressive, inexorable loss of cognitive function associated with an excessive number of senile plaques in the cerebral cortex and subcortical gray matter, which also contains β-amyloid and neurofibrillary tangles consisting of tau protein" (Merck Manual 2004). Alzheimer's disease is about twice as common in women as in men, and it accounts for > 65 % of dementia cases in the elderly. Vascular dementia and Alzheimer's disease coexist in about 15 % of cases.

Alzheimer's disease is believed to represent the fourth most common medical cause of death. Prevalence of the disease doubles every 5 years beyond age 65 (National Institute on Aging: Prevalence and costs of Alzheimer's disease. Progress Report on Alzheimer's Disease. NIH Publication No. 99 3616, November 1998; Polvikoski et al., Neurology, 2001, 56:1690-1696). Alzheimer's disease currently affects about 15 million people world-wide (including all races and ethnic groups) and, due to the relative increase of elderly people in the population, its prevalence is likely to increase over the next two to three decades.

At present, Alzheimer's disease cannot be cured, i.e. there is no treatment available which effectively reverses its symptoms and progression. Some drugs may, however, alleviate certain symptoms associated with the disease and reduce dependency on care. Among the first drugs approved for the treatment of Alzheimer's disease are members of the class of cholinesterase inhibitors, such as donepezil, galantamine, and tacrine.

Memantine, and pharmaceutically acceptable salts thereof (*e*.*g*., the HCl salt), is approved in the U.S. for treatment of Alzheimer's disease. Memantine is currently approved outside the United States as an oral formulation for both Alzheimer's and Parkinson's Disease. Memantine (1-amino-3,5-dimethyladamantane, disclosed in, *e*.*g*., U.S. Patent Nos. 4,122,193; 4,273,774; 5,061,703), is a systemically-active uncompetitive NMDA receptor antagonist having low to moderate affinity for the receptor and strong voltage dependency and rapid blocking/unblocking kinetics. These pharmacological features allow memantine to block sustained activation of the receptor under pathological conditions and to rapidly leave the NMDA channel during normal physiological activation of the channel.

In the EU, memantine is available in the form of a film - coated tablet containing 10 mg Memantine hydrochloride (Axura^{®} Film - coated tablets, marketed by Merz Pharmaceutical GmbH).

In some European countries, memantine is also available as liquid solution for oral use. For example, in Germany a liquid formulation (Axura^{®} drops) with a strength of 10 mg/g is marketed by Merz Pharmaceuticals GmbH. This liquid formulation has two major advantages over tablets. It allows flexible dosing and it does not require the swallowing of solid dosage units, which is difficult for many elderly Alzheimer's patients. Flexible dosing may be recommended during therapy, when a dose or dosing scheme may be individually adapted for each patient, depending on the state or progression of the disease. In order to achieve dose reduction for tablet administration, the tablet must be divided in halves, quarters or even more subparts, which may be difficult for some patients.

Liquid formulations containing memantine and its salts also have certain disadvantages. Since liquid formulations comprise a drug substance in an already dissolved form, the taste and the smell of the compound will be more readily noticed than in the case of solid dosage forms like e.g. a film-coated tablet. The taste of memantine is particularly poor, e.g., it is bitter and has an astringent effect on the oral mucosa. Such undesirable properties are usually experienced by a patient in a more pronounced manner if the drug is formulated as a liquid formulation than if the drug is formulated as a film coated tablet or capsule. Moreover, while encapsulation is a common taste-masking technique in solid dosage forms, it is technically very difficult to encapsulate drug substance in a liquid formulation, particularly in the case of compounds having substantial water-solubility. Similarly, a compound having an unpleasant smell may also be difficult to formulate into a palatable liquid preparation. Depending on the intensity of the taste and/or smell of the compound, it may not be sufficient to rely on the incorporation of sweeteners and flavouring agents in order to develop an acceptable aqueous or semi-solid formulation.

Conventional aqueous liquid formulations may have other disadvantages, including their potential to enhance the chemical degradation of the drug substance, e.g. by hydrolysis, and thus to lead to a decreased shelf life of the drug product. Moreover, the microbiological stability of an aqueous liquid formulation is typically inferior to that of a solid dosage form such as a tablet or capsule, and, therefore it is often necessary to incorporate preservatives into aqueous liquid formulations.

1-aminoadamantanes, such as memantine and pharmaceutically acceptable salts thereof, exhibit taste and smell properties which are problematic and limit the degree of acceptability of conventional liquid or semi-solid formulations, and some patients who might otherwise benefit from liquid formulations may prefer to take these drugs in tablet form.

Thus, a need exists for liquid and/or semi-solid formulations comprising 1-aminoadamantane derivatives, such as memantine and pharmaceutically acceptable salts thereof, with improved organoleptic properties like smell and taste.

The instant inventors have discovered that compositions comprising a 1-aminoadamantane derivative (including memantine and pharmaceutically acceptable salts thereof) and one or more pharmaceutically acceptable cyclodextrins do not exhibit the disadvantages typically associated with aqueous liquid dosage forms and that such compositions are palatable and acceptable to a larger number of patients due to their improved organoleptic properties.

### OBJECTS OF THE INVENTION

It is an object of the invention to provide compositions comprising a 1-aminoadamantane derivative, such as memantine and pharmaceutically acceptable salts thereof, and a pharmaceutically acceptable cyclodextrin or a combination of pharmaceutically acceptable cyclodextrins, which compositions exhibit a neutral or sweet taste and little or no unpleasant smell and are remarkably palatable. An additional object of the invention is to provide a water-soluble complex of an 1-aminoadamantane derivative, such as memantine and pharmaceutically acceptable salts thereof, and a pharmaceutically acceptable cyclodextrin and/or cyclodextrin derivative, which complex may be incorporated into a solid, semi-solid or particularly liquid formulations for oral administration which are chemically and microbiologically stable and have a remarkable degree of palatability. It is a further object of the invention to employ such compositions in the treatment of CNS disorders, including Alzheimer's disease. Yet additional objects will become apparent hereinafter, and still further objects will be apparent to one skilled in the art.

### SUMMARY OF THE INVENTION

The present invention relates to a composition comprising a 1-aminoadamantane derivative selected from those of formula (I) 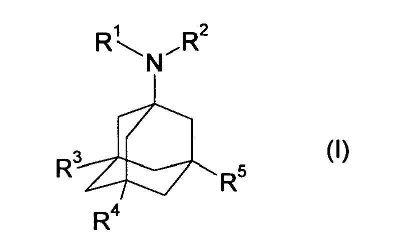 wherein
R¹ and R², which may be the same or different, represent hydrogen or C₁₋₆alkyl, or together represent -(CH₂)ₓ-, wherein x is 2 to 5, inclusive;
R³ and R⁴, which may be the same or different, represent hydrogen, C₁₋₆alkyl, cycloC₅₋₆alkyl, or phenyl; and
R⁵ represents hydrogen or C₁₋₆alkyl, and optical isomers, enantiomers, hydrates, solvates, polymorphs, and pharmaceutically acceptable salts thereof;
and a pharmaceutically acceptable cyclodextrin or combination of pharmaceutically acceptable cyclodextrins.

A further aspect of the invention relates to such a composition wherein the pharmaceutically acceptable cyclodextrin is selected from pharmaceutically acceptable, water-soluble, native or derivatised cyclodextrins, such as such as alpha-cyclodextrin, beta-cyclodextrin, randomly methylated beta-cyclodextrin, 2-O-methyl-beta-cyclodextrin, heptakis-(2,6-di-O-methyl)-beta-cyclodextrin (dimethyl-beta-cyclodextrin), acetylated dimethyl-beta-cyclodextrin, heptakis-(2,3,6-tri-O-methyl)-beta-cyclodextrin (trimethyl-beta-cyclodextrin, sulfoalkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclodextrin, maltosyl-beta-cyclodextrin, beta-cyclodextrin sulphate, beta-cyclodextrin phosphate, gamma-cyclodextrin, sulfoalkylether-beta-cyclodextrin, and sulfobutylether-beta-cyclodextrin, and hydroxyalkyl-modified cyclodextrins (including hydroxypropyl-beta-cyclodextrin, e.g., 2-hydroxypropyl-beta-cyclodextrin, or hydroxypropyl-gamma-cyclodextrin, e.g., 2-hydroxypropyl-gamma-cyclodextrin).

A further aspect of the invention relates to such a composition wherein the molar ratio of the cyclodextrin to the 1-aminoadamantane derivative is a maximum of 50 : 1, (including compositions wherein the molar ratio is 10 : 1 or 9 : 1 or 8 : 1 or 7 : 1 or 6 : 1 or 5:1 or 4:1 or 3:1 or 2:1 or 1:1) and is at least about 0.1 : 1.

A further aspect of the invention relates to such a composition in the form of an aqueous liquid composition.

A further aspect of the invention relates to such a composition in the form of a semi-solid composition including a gel, a cream or an ointment with an acceptable viscosity.

A further aspect of the invention relates to such a composition, wherein the composition is a rapidly or very rapidly dissolving solid composition, e.g. a powder, a granule or a lyophilisate.

A further aspect of the inventions relates to such a composition, wherein the composition is a rapidly or very rapidly dissolving water soluble powder, a granule or a lyophilisate.

A further aspect of the invention relates to a formulation for reconstitution in form of a powder, granules or a lyophilisate (including such a composition wherein reconsitution of the powder or lyophilisate with an aqueous solvent results in an aqueous liquid composition).

An further aspect of the invention relates to such a composition wherein the pharmaceutically acceptable cyclodextrin is selected from pharmaceutically acceptable, water-soluble, native or derivatised cyclodextrins, such as such as alpha-cyclodextrin, beta-cyclodextrin, randomly methylated beta-cyclodextrin, 2-O-methyl-beta-cyclodextrin, heptakis-(2,6-di-O-methyl)-beta-cyclodextrin (dimethyl-beta-cyclodextrin), acetylated dimethyl-beta-cyclodextrin, heptakis-(2,3,6-tri-O-methyl)-beta-cyclodextrin (trimethyl-beta-cyclodextrin, sulfoalkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclodextrin, maltosyl-beta-cyclodextrin, beta-cyclodextrin sulphate, beta-cyclodextrin phosphate, gamma-cyclodextrin, sulfoalkylether-beta-cyclodextrin, and sulfobutylether-beta-cyclodextrin, and hydroxyalkyl-modified cyclodextrins (including hydroxypropyl-beta-cyclodextrin, e.g., 2-hydroxypropyl-beta-cyclodextrin, or hydroxypropyl-gamma-cyclodextrin, e.g., 2-hydroxypropyl-gamma-cyclodextrin).

An additional aspect of the invention relates to such a composition wherein the compound of formula (I) is memantine or a pharmaceutically acceptable salt thereof.

A further aspect of the invention relates to such a composition wherein the concentration of memantine or a pharmaceutically acceptable salt thereof is in the range from about 1 mg/ml to about 100 mg/ml (including from about 2 mg/ml to about 75 mg/ml and from about 5 mg/ml to about 50 mg/ml).

An additional aspect of the invention relates to any of the above-described compositions wherein the composition further comprises at least one flavouring agent or taste-masking agent other than a cyclodextrin.

An additional aspect of the invention relates to any of the above-described compositions wherein the composition is substantially free of preservatives.

An additional aspect of the invention relates to any of the above-described compositions wherein the composition further comprises a preservative and the concentration of the preservative is below the concentration needed to effectively preserve an equivalent placebo composition.

An additional aspect of the invention relates to any of the above-described compositions wherein the composition further comprises at least one additional active ingredient.

A further aspect of the invention relates to a pharmaceutical composition comprising any of the above-described compositions in combination with one or more additional pharmaceutically acceptable excipients.

An additional aspect of the invention relates to such a pharmaceutical composition in the form of an aqueous liquid composition.

An additional aspect of the invention relates to such a pharmaceutical composition in the form of a semi-solid composition.

An additional aspect of the invention relates to such a composition in the form of a solid composition including rapidly dissolving water - soluble powder and granule.

A further aspect of the invention relates to a medicament comprising any of the above-described compositions.

A further aspect of the invention relates to the use of the above-described compositions in treating CNS disorders, including neurodegenerative disorders, Alzheimer's disease, Parkinson's disease, AIDS dementia, neuropathic pain, cerebral ischemia, epilepsy, hepatic encephalopathy, multiple sclerosis, stroke, depression, tardive dyskinesia, autism, ADHD, and autistic spectrum disorders.

A further aspect of the invention relates to a method of treating CNS disorders, including neurodegenerative disorders, Alzheimer's disease, Parkinson's disease, AIDS dementia, neuropathic pain, cerebral ischemia, epilepsy, hepatic encephalopathy, multiple sclerosis, stroke, depression, tardive dyskinesia, autism, ADHD, and autistic spectrum disorders in a subject in need thereof, comprising administering an effective amount of a composition as described above.

Non-limiting examples of the 1-aminoadamantane derivatives of formula (I) used according to the present invention include:
1-amino-3-phenyl adamantane,
1-amino-methyl adamantane,
1-amino-3,5-dimethyl adamantane (memantine),
1-amino-3-ethyl adamantane,
1-amino-3-isopropyl adamantane,
1-amino-3-n-butyl adamantane,
1-amino-3,5-diethyl adamantane,
1-amino-3,5-diisopropyl adamantane,
1-amino-3,5-di-n-butyl adamantane,
1-amino-3-methyl-5-ethyl adamantane,
1-N-methylamino-3,5-dimethyl adamantane,
1-N-ethylamino-3,5-dimethyl adamantane,
1-N-isopropyl-amino-3,5-dimethyl adamantane,
1-N,N-dimethyl-amino-3,5-dimethyl adamantane,
1-N-methyl-N-isopropyl-amino-3-methyl-5-ethyl adamantane,
1-amino-3-butyl-5-phenyl adamantane,
1-amino-3-pentyl adamantane,
1-amino-3,5-dipentyl adamantane,
1-amino-3-pentyl-5-hexyl adamantane,
1-amino-3-pentyl-5-cyclohexyl adamantane,
1-amino-3-pentyl-5-phenyl adamantane,
1-amino-3-hexyl adamantane,
1-amino-3,5-dihexyl adamantane,
1-amino-3-hexyl-5-cyclohexyl adamantane,
1-amino-3-hexyl-5-phenyl adamantane,
1-amino-3-cyclohexyl adamantane,
1-amino-3,5-dicyclohexyl adamantane,
1-amino-3-cyclohexyl-5-phenyl adamantane,
1-amino-3,5-diphenyl adamantane,
1-amino-3,5,7-trimethyl adamantane,
1-amino-3,5-dimethyl-7-ethyl adamantane,
1-amino-3,5-diethyl-7-methyl adamantane,
1-N-pyrrolidino and 1-N-piperidine derivatives,
1-amino-3-methyl-5-propyl adamantane,
1-amino-3-methyl-5-butyl adamantane,
1-amino-3-methyl-5-pentyl adamantane,
1-amino-3-methyl-5-hexyl adamantane,
1-amino-3-methyl-5-cyclohexyl adamantane,
1-amino-3-methyl-5-phenyl adamantane,
1-amino-3-ethyl-5-propyl adamantane,
1-amino-3-ethyl-5-butyl adamantane,
1-amino-3-ethyl-5-pentyl adamantane,
1-amino-3-ethyl-5-hexyl adamantane,
1-amino-3-ethyl-5-cyclohexyl adamantane,
1-amino-3-ethyl-5-phenyl adamantane,
1-amino-3-propyl-5-butyl adamantane,
1-amino-3-propyl-5-pentyl adamantane,
1-amino-3-propyl-5-hexyl adamantane,
1-amino-3-propyl-5-cyclohexyl adamantane,
1-amino-3-propyl-5-phenyl adamantane,
1-amino-3-butyl-5-pentyl adamantane,
1-amino-3-butyl-5-hexyl adamantane,
1-amino-3-butyl-5-cyclohexyl adamantane,
and optical isomers, diastereomers, enantiomers, hydrates, their pharmaceutically acceptable salts, and mixtures thereof.

### DETAILED DESCRIPTION OF THE INVENTION

As used herein, the term aqueous liquid composition means a liquid preparation wherein the major liquid component is water. Optionally, the aqueous liquid composition may further comprise other liquid components, such as pharmaceutically acceptable organic solvents and cosolvents. Examples of such other liquid components are e.g. ethanol, glycerol, propylene glycol, and polyethylene glycol. Such water-miscible organic solvents and cosolvents may be incorporated for example in order to solubilise a poorly water-soluble ingredient, such as a lipophilic substance (e.g. memantine base).

The term liquid formulation includes liquid solutions and dispersions, such as emulsions and suspensions.

As used herein, the term semi-solid composition means a preparation with low viscosity whose major liquid component is water. Optionally, the semi-solid composition may further comprise other liquid components, such as pharmaceutically acceptable organic solvents, cosolvents, viscosity regulation polymers and emulsifiers. Examples of such other liquid components are ethanol, glycerol, propylene glycol, and polyethylene glycol. Such water-miscible organic solvents may be incorporated for example in order to solubilise a poorly water-soluble ingredient, such as a lipophilic substance.

The term semi-solid composition includes gels, creams and ointments. In comparison to a liquid composition these formulations are not freely flowing. The viscosity of semi-solid compositions may be controlled by one polymer or by a combination of polymers.such as acacia gum and derivatives, alginic acid and derivatives, carbomer and derivatives, carboxymethylcellulose and derivatives, carrageenan and derivatives, croscarmellose and derivatives, crospovidone and derivatives, dextrin and derivatives, ethylcellulose and derivatives, gelatin and derivatives, guar gum and derivatives, hydroxyethyl cellulose and derivatives, hypromellose and derivatives, hydroxypropyl methylcellulose and derivatives, lecithin and derivatives, maltodextrin and derivatives, methylcellulose and derivatives, poloxamer and derivatives, polethylenglycoles and derivatives, polymethacrylates and derivatives, polyoxyethylalkylethers and derivatives, polyvinylalkohol, polyvinylpyrrolidon and derivatives, silicon dioxide and derivatives, sodium starch glycolate and derivatives, sorbitol and derivatives, starch and derivartives, pregelatinised starch and derivatives, tragacanth and derivatives and xanthan gum and derivatives,

1-Aminoadamantane derivatives of formula (I) (e.g., memantine, 1-amino-3,5-dimethyladamantane) are disclosed in U.S. Patent No. 5,061,703. Compounds of formula (I) (e.g., memantine) may be used according to the invention in the form of any of pharmaceutically acceptable salts, solvates, isomers, conjugates, and prodrugs, any references to compounds of formula (I) (e.g., memantine) in this description should be understood as also referring to such salts, solvates, isomers, conjugates, and prodrugs.

The term "analog" or "derivative" is used herein in the conventional pharmaceutical sense, to refer to a molecule that structurally resembles a reference molecule (such as memantine), but has been modified in a targeted and controlled manner to replace one or more specific substituents of the referent molecule with an alternate substituent, thereby generating a molecule which is structurally similar to the reference molecule. Synthesis and screening of analogs (*e*.*g*., using structural and/or biochemical analysis), to identify slightly modified versions of a known compound which may have improved or biased traits (such as higher potency and/or selectivity at a specific targeted receptor type, greater ability to penetrate mammalian blood-brain barriers, fewer side effects, etc.) is a drug design approach that is well known in pharmaceutical chemistry.

Pharmaceutically acceptable salts include, but are not limited to, acid addition salts, such as those made with hydrochloric, methylsulfonic, hydrobromic, hydroiodic, perchloric, sulfuric, nitric, phosphoric, acetic, propionic, glycolic, lactic, pyruvic, malonic, succinic, fumaric, tartaric, citric, benzoic, carbonic, cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid. All of these salts (or other similar salts) may be prepared by conventional means. The nature of the salt is not critical, provided that it is non-toxic and does not substantially interfere with the desired pharmacological activity.

Cyclodextrins are oligosaccharides composed of glucopyranose units. The major unsubstituted or native cyclodextrins are usually prepared by the enzymatic degradation of starch. They are cone-like, toroidal molecules with a rigid structure and a central cavity, the size of which varies according to the cyclodextrin type. The internal cavity of cyclodextrins is more hydrophobic and capable of accommodating both lipophilic and hydrophilic molecules in the form of inclusion complexes, thus enabling, for example, the solubilisation of poorly soluble drugs in aqueous media. Other types of complexes involving cyclodextrins have recently been identified.

As used herein, a complex means an association of molecules by non-covalent interactions. Complexation taking place in solution is typically an equilibrium process. However, complexes may also occur in solid state. An inclusion complex is a structure wherein a guest molecule is either partially or completely contained within a cavity of a larger host molecule.

The three major types of pharmaceutically acceptable cyclodextrins are alpha-, beta- and gamma-cyclodextrins, comprising 6, 7 and 8 glucopyranose units, respectively. In addition, a number of chemically modified cyclodextrins have been developed, mostly motivated by the desire to increase the water solubility and extend their usefulness as solubilising excipients. Examples of such derivatives include alpha-cyclodextrin, beta-cyclodextrin, randomly methylated beta-cyclodextrin, 2-O-methyl-beta-cyclodextrin, heptakis-(2,6-di-O-methyl)-beta-cyclodextrin (dimethyl-beta-cyclodextrin), acetylated dimethyl-beta-cyclodextrin, heptakis-(2,3,6-tri-O-methyl)-beta-cyclodextrin (trimethyl-beta-cyclodextrin, sulfoalkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclodextrin, maltosyl-beta-cyclodextrin, beta-cyclodextrin sulphate, beta-cyclodextrin phosphate, gamma-cyclodextrin, sulfoalkylether-beta-cyclodextrin, and sulfobutylether-beta-cyclodextrin (SBEBCD), and hydroxyalkyl-modified cyclodextrins (including hydroxypropyl-beta-cyclodextrin, e.g., 2-hydroxypropyl-beta-cyclodextrin (HPBCD), or hydroxypropyl-gamma-cyclodextrin, e.g., 2-hydroxypropyl-gamma-cyclodextrin (HPGCD)). As used herein, the term "cyclodextrins" includes such modified versions of the native cyclodextrins.

An example of a suitable grade of hydroxypropyl-beta-cyclodextrin is amorphous, randomly substituted hydroxypropyl-beta-cyclodextrin with a degree of substitution DS in the range of about 4.5 (i.e. between approx. 4 and 5), such as the product marketed as Kleptose↗ HPB (Roquette). It is noted that the DS value, as used herein, defines the average number of substituted hydroxyl groups per anhydroglucose unit, not per cyclodextrin molecule. Other examples of useful grades are randomly substituted hydroxypropyl-beta-cyclodextrin with a degree of substitution DS in the range of about 5.6, or in the range of 2 to 4, or in the range of 5, or in the range of 6.5, respectively. An example of a suitable grade of hydroxypropyl-gamma-cyclodextrin is the product marketed as Cavasol↗ W8 HP (Wacker Chemie).

The amount of cyclodextrin in the composition may be selected taking into account the type of cyclodextrin and the concentration of the active ingredient which is to be achieved. The concentration of the active ingredient may be at least about 1 mg/ml. If the active ingredient is memantine or memantine hydrochloride, the concentration of the drug substance in the liquid composition may be in the range from about 1 to about 100 mg/ml. Alternatively, the pharmaceutical compositions may comprise memantine in a range of 2 to 50 mg/ml. For example, a concentration of 10 mg/ml of memantine hydrochloride may be formulated in such a composition.

Compositions according to the present invention may be obtained when the amount of the cyclodextrin is selected to give a molar cyclodextrin-to-active ratio of at least about 0.1 : 1 or at least 1 : 1. Other embodiments may comprise a molar cyclodextrin-to-active ratio of at least about 0.1 : 1 to a maximum of 100 : 1. Other embodiments may comprise a ratio of about 1 : 1 to 50 : 1. The molar ratio of the cyclodextrin to the drug substance may also be formulated from about 1 : 1 to about 10 : 1, or from about 2 : 1 to about 10 : 1 such as about 3 : 1, or about 6 : 1, or about 9 : 1, respectively.

It has been found that such ratios lead to a remarkably high degree of taste masking, which may be associated with the spontaneous formation of a soluble complex between the cyclodextrin molecule and the drug substance. Moreover, the unpleasant smell of the active compound, in particular in the case of a memantine compound, is substantially reduced. The degree of taste-masking is surprising in view of the high water-solubility of the active compounds, particularly in the case of readily soluble salt forms, such as memantine hydrochloride. It is also surprising that the complexation achieves effective taste-masking without eliminating other properties of the drug substance which are believed to result from their state of being dissolved in the aqueous solvent. After oral administration, for example, the active compound is rapidly absorbed from the composition and becomes bioavailable without any delay.

If the molar ratio of the cyclodextrin to the active compound is selected to provide substantial but not complete taste masking, which may occur if the ratio is chosen in the lower part of the ranges given above (e.g., a ratio of 1 : 1), the addition of one or more further excipients which improve the palatability of the formulation may also be contemplated. This is particularly true for memantine hydrochloride solutions in the preferred drug concentration range. For example, one or more sweeteners may be incorporated. Furthermore, one or more excipients selected from the group of flavours, flavour enhancers, and taste masking agents may be added, with the proviso that such taste-masking agents are not selected from the class of cyclodextrins.

Sweeteners, as used herein, are natural or synthetic compounds which have a sweet taste and are physiologically acceptable. Examples of natural sweeteners include common sugars and sugar alcohols such as sucrose, glucose, fructose, maltose, maltitol, xylitol, lactitol, mannitol, and sorbitol. A sugar alcohol may be used to improve the flavour of the composition of the invention, for example sorbitol. A useful concentration range for sorbitol or other sugars and sugar alcohols is from about 5 % (w/v) to about 25 % (w/v), a 10 % (w/v) may also be used.

In another embodiment, an artificial sweetener is incorporated in the composition in addition to, or instead of, a natural sweetener. Useful artificial sweeteners include saccharin-sodium, saccharin, sodium cyclamate, acesulfame K, neohesperidine dihydrochalcone, and aspartame, as well as any other sweeteners whose safety in human use is established. Appropriate concentrations depend on the individual sweetener which is selected, but also on the specific cyclodextrin which is chosen. For example, hydroxypropyl-beta-cyclodextrin already provides for a rather sweet taste so that the addition of a sweetening agent may not increase the palatability of the formulation any further.

Suitable flavors which may further improve the taste of cyclodextrin-containing aqueous compositions of aminoadamantane derivatives (including memantine and pharmaceutically acceptable salts thereof) include grape, orange, peppermint, spearmint, cherry, liquorice, and aniseed. In particular, peppermint flavours are physicochemically and organoleptically well-compatible with the key components of the composition of the invention and may lead to palatable formulations.

If an oily flavouring component (such as peppermint oil) is used, it may be necessary or useful to also add an excipient which can contribute to the solubilisation of the oil in the aqueous phase. For example, a surfactant or a water-miscible organic cosolvent may be used for this purpose. In the case of peppermint oil or other peppermint flavouring products, solubilisation may be achieved by the incorporation of propylene glycol.

For liquid compositions it may be useful that the complex of the invention leads to a strong taste-masking effect while not inhibiting the preservative effect of the active ingredient. As a result of the retained antimicrobial effect of the drug substance, it may be possible to formulate the composition of the invention without any additional preservative. In one of the embodiments, therefore, the composition of the invention is indeed substantially free of preservatives. In this context, the term "substantially" means that preservatives are not detectable in the composition, or only in concentrations which are generally considered irrelevant with regard to any preservation effects.

The liquid composition may, optionally, comprise at least one preservative, but at a concentration which is insufficient to effectively preserve an equivalent placebo composition. As used herein, an equivalent placebo composition is defined as a composition, substantially free of active ingredients, whose properties and other ingredients are largely the same as those of the drug-containing reference composition

Whether a composition is effectively preserved may be determined according to tests known to those skilled in the art, such as the test for preservative efficacy (USP <51>), wherein five challenge organisms are added to test compositions at defined time intervals, depending on the product category. Conducted in appropriate series, such testing can also be performed in order to determine the minimally effective concentration of a specific preservative for a given composition, such as a drug-free composition equivalent to a composition according to the invention. For example, it may be found that in order to effectively preserve a particular placebo composition with sorbic acid, the preservative must be present at a concentration of at least about 0.1 % (w/v). In this case, the reference composition which comprises the 1-aminoadamantane derivative could contain sorbic acid at a substantially lower concentration, such as about 0.05 % (w/v) or less. In another embodiment, the concentration of the preservative is selected to be not more than about a fifth, including not more than about a tenth, of the concentration needed to effectively preserve an equivalent placebo composition.

For reproducible product quality and reliable stability, the composition may be adjusted to a specific pH by incorporating one or more appropriate excipients selected from the group consisting of physiologically acceptable acids, bases, and acidic and alkaline salts. For example, the combination of citric acid and sodium citrate may be used for buffering the pH of the composition at a value selected in the range from about pH 4 to about pH 10. In particular, the pH may be adjusted to a value from about pH 4.5 to about pH 8, using pharmaceutically acceptable buffering agents or -mixtures such as citrate buffer.

Further excipients which are routinely used in pharmaceutical formulations may be incorporated as may seem appropriate to adjust the composition to the specific requirements of a particular drug candidate, or to a specific use or target population. Examples of potentially suitable excipients are thickeners such as soluble gums including carrageenan, alginate, xanthan, and soluble cellulose esters; colouring agents; stabilizers, such as antioxidants, or crystallization inhibitors, such as glycerol, propylene glycol, or polyvinylpyrrolidone.

Optionally, the composition may further comprise another active ingredient which is not an aminoadamantane derivative. As used herein, an active ingredient is a pharmaceutically acceptable compound or mixture of compounds useful for the diagnosis, prevention, or treatment of a symptom, disease, or condition. The terms "active compound", "active ingredient", "drug", and "drug substance" may be used interchangeably. Additional active ingredients include acetylcholinesterase inhibitors, (such as donepezil, rivastigmine, tacrine, galantamine, physostigmine, huperzine A, zanapezil, ganstigmine, phenserine, phenethylnorcymserine (PENC), cymserine, thiacymserine, SPH 1371 (galantamine plus), ER 127528, RS 1259, and F3796), antipsychotics (including atypical antipsychotics such as olanzapine, clozapine, risperidone, sertindole, quetiapine, ziprasidone, surmontill and aripiprazole and typical antipsychotics such as acepromazine, benperidol, bromazepam, bromperidol, chlorpromazine, chlorprothixene, clotiapine, cyamemazine, diazepam, dixyrazine, droperidol, flupentixol, fluphenazine, fluspirilene, haloperidol, heptaminol, isopropamide iodide, levomepromazine, levosulpiride, loxapine, melperone, mesoridazine, molindone, oxypertine, oxyprothepine, penfluridol, perazine, periciazine, perphenazine, pimozide, pipamperone, pipotiazine, prochlorperazine, promazine, promethazine, prothipendyl, pyridoxine, sulpiride, sultopride, tetrabenazine, thioproperazine, thioridazine, tiapride, tiotixene, trifluoperazine, triflupromazine, trihexyphenidyl, and zuclopenthixol), antidepressants (including selective serotonin reuptake inhibitors (SSRIs) such as citalopram and escitalopram, sertraline, bupropion, reboxetine, amirtazapine, fluvoxamine, milnacipran, venlafaxine, duloxetine, mirtazapine and atomoxetine), glitazones (such as pioglitazone, troglitazone, rosiglitazone, ciglitazone, englitazone, darglitazone, rivoglitazone, isaglitazone, KRP 297, T 174, and NP 0110), dextromethorphan, and dopamine antagonists such as trans-1-{4-[2-[4-(2,3-dichlorophenyl)-piperazin-1-yl]-ethyl]-cyclohexyl}-3,3-dimethyl-urea, or a pharmaceutically acceptable salt thereof).

It has been surprisingly found that the complex between memantine and its pharmaceutically acceptable salts and one cyclodextrin or a combination of cyclodextrins is formed spontaneously in aqueous media. The complex formation takes place with all types of native cyclodextrins and cyclodextrin derivatives, including beta- and gamma-cyclodextrin as well as hydroxypropyl-beta- or hydroxypropyl -gamma-cyclodextrin.

The complex formation takes place spontaneously in aqueous solutions at normal room temperature, which is in contrast to many other known cyclodextrin complexes whose preparation requires the application of heat and/or very long stirring times. Hence, the preparation of the composition is technically easy, quick and cost-efficient. In most cases, the components are simply weighed and combined with a measured amount of water or cosolvents followed by stirring until dissolution occurs. The mixture may be agitated and/or heated. The solution may be further processed by filtration or centrifugation to remove residual particles. If a solid complex is desired, the solution may be dried, such as by spray drying or freeze drying.

The complex formation may also be achieved by conventional single pot wet granulation processes or fluid bed granulation processes using only very limited amounts of aqueous media or organic solvents or cosolvents or mixtures of them. The complex can also be formed by spray drying the memantine cyclodextrin solution. For the conventional granulation processes memantine and the cyclodextrin can be blended homogeneously and then be granulated with an aqueous solution or a cosolvent containing aqueous solution. The granulation process can also be performed in that way, that memantine is granulated with a cyclodextrin solution or that a cyclodextrin powder is granulated with a memantine solution. Only in the case of adding a water-insoluble ingredient it may be useful to first dissolve that excipient in an amount of surfactant or cosolvent before combining it with the remaining components.

In general, various methods may be used to make such cyclodextrin/drug complex, such as the solution method, the co-precipitation method, the slurry method, the kneading method and the grinding method (T Loftsson, Pharmaceutical Technology 12, 41-50, 1999). The slurry method and the kneading method are more often used for the complexation of poorly water-soluble compounds, whereas the solution and the precipitation method may easily be used for both water-soluble and poorly-soluble compounds.

The present liquid and semi-solid compositions may be filled into a container which holds a plurality of doses. Appropriate containers will hold a volume in the range from about 5 ml or 5 g to about 1,000 ml or 1,000 g, and other containers may hold from about 10 ml (or g) to about 500 ml (or g). The volume is selected in consideration of the strength of the specific formulation and the time period for which the product is to be used. For example, a container may be selected to accommodate the medication needed for several days, weeks, or months. In one of the preferred embodiments, the container is selected to hold sufficient medication for at least about 4 weeks. In another embodiment, the container is selected to hold about 50 ml (or g), about 100 ml (or g), about 200 ml (or g), about 250 mi (or g), or about 500 ml (or g).

Appropriate containers may be of glass or a suitable plastic, such as polypropylene or polyethylene, and will usually have a closure which is reclosable. Optionally, the closure system is child-proof.

The container may further comprise a means for measuring and/or dispensing defined doses of the composition. A conventional measuring means is, for example, a dropper, i.e. a glass tube fitted with a rubber bulb which is integrated in the closure and removed when opening the container. Alternatively, a non-removable dropper may be integrated in the bottle neck.

The container, or container system, may also comprise a dosing cup that provides markings indicating the amount of liquid to be taken for the most common doses. For example, the markings may range from about 0.5 ml to about 10 ml, and from about 1 ml to about 5 ml, or instead of volumes they may indicate the dose in grams of formulation, or in mg of drug substance. The measuring cup may be part of the container closure system, or it may be provided as a separate device within the secondary package in which the container is presented.

The composition of the present invention may be in the form of a preparation for reconstitution, such as a powder, a granulate, a tablet, a wafer or a lyophilisate for the preparation of a ready-to-use liquid or semi solid formulation for oral administration. In this case, the composition may be composed in the same manner as described above in the context of a liquid composition in view of the same effects and advantages, except that it does not comprise a liquid. Instead, such product is designed to be combined with a liquid, such as water or a mixture of water and a cosolvent, before administration. In comparison with a ready-to-use liquid composition, such solid composition for reconstitution is potentially more stable and may exhibit a longer shelf-life. It is also lighter and less costly to ship and store; however, a ready-to-use liquid formulation may be more convenient for those patients who prefer not to have to manipulate the formulation before administration.

A rapidly dissolving solid composition for reconstitution may be prepared by conventional pharmaceutical processing steps. For example, it may be prepared in a similar manner as the liquid composition described above, and subsequently dried, e.g. by lyophilisation or spray drying.

Alternatively, the solid composition may be designed and formulated for oral administration as such, without reconstitution. In this context, oral administration is understood as comprising all forms of oral use, including intraoral and peroral administration. An example of dosage forms for intraoral administration are those formulations which disintegrate in the mouth rather than in the gastric fluid, which formulations are also known as orally disintegrating dosage forms, fast-melt tablets and oral wafers. Examples of formulations for peroral administration include conventional hard or soft capsules and tablets.

As used herein in conjunction with the compositions of the present invention, the term "rapidly dissolving" means the release of a 1-aminocyclohexane derivative (e.g., neramexane or a pharmaceutically acceptable salt thereof) of at least 85% within 30 minutes, as determined using a paddle (50 rpm) or basket (100 rpm) apparatus at a temperature of about 37 °C and a volume of about 900 ml, and the term "very rapidly dissolving" means the release of a 1-aminocyclohexane derivative (e.g., neramexane or a pharmaceutically acceptable salt thereof) of at least 85% within 15 minutes, as determined using a paddle (50 rpm) or basket (100 rpm) apparatus at a temperature of about 37 °C and a volume of about 900 ml.

As used herein, the term medicament includes a drug product which may represent the composition as such or in combination with a container or other packaging means, a dosing device, a liquid for reconstitution, or with another drug substance-containing composition in the form of a kit or combination package.

According to some embodiments, the present invention relates to administration of compositions comprising a 1-aminoadamantane derivative (including memantine and pharmaceutically acceptable salts thereof), to an individual in need thereof. For example, the compositions of the invention are suitable for the treatment of CNS disorders, including but not limited to the treatment of neurodegenerative disorders, Alzheimer's disease, Parkinson's disease, AIDS dementia (U.S. Patent Nos. 5,506,231, 5,061,703, and 5,614,560; see also Parsons et al., Neuropharmacology 1999 June; 38(6):735-67), neuropathic pain (U.S. Patent No. 5,334,618), cerebral ischemia (U.S. Patent No. 5,061,703), epilepsy, hepatic encephalopathy, multiple sclerosis, stroke, depression (U.S. Patent No. 6,479,553), and tardive dyskinesia.

1-aminoadamantane derivatives, including memantine and pharmaceutically acceptable salts thereof, may also be effective for the treatment of autism, ADHD and other autistic spectrum disorders (U.S. Published Application No. 2006/0079582).

The term "treat" is used herein to mean to relieve or alleviate at least one symptom of a disease in a subject. Within the meaning of the present invention, the term "treat" also denotes to arrest, delay the onset (*i.e*., the period prior to clinical manifestation of a disease) and/or reduce the risk of developing or worsening a disease.

The term "therapeutically effective" applied to dose or amount refers to that quantity of a compound or pharmaceutical composition that is sufficient to result in a desired activity upon administration to a mammal in need thereof.

The phrase "pharmaceutically acceptable", as used in connection with compositions of the invention, refers to molecular entities and other ingredients of such compositions that are physiologically tolerable and do not typically produce untoward reactions when administered to a mammal (*e*.*g*., human). The term "pharmaceutically acceptable" may also mean approved by a regulatory agency of the Federal or a state government or listed in the U.S. Pharmacopeia or other generally recognized pharmacopeia for use in mammals, and more particularly in humans.

The term "about" or "approximately" usually means within 20%, alternatively within 10%, including within 5% of a given value or range. Alternatively, especially in biological systems, the term "about" means within about a log (*i*.*e*., an order of magnitude), including within a factor of two of a given value.

The compositions of the present invention may be used for the manufacture of a medicament for the treatment of at least one of the mentioned disorders, wherein the medicament is adapted to or appropriately prepared for a specific administration as disclosed herein (e.g., to once-a-day, twice-a-day administration, or three times a day administration). For this purpose the package leaflet and/or the patient information contains corresponding information.

### EXAMPLES

The following examples illustrate the invention without limiting its scope.

### Example 1

Complexes between memantine hydrochloride and either native cyclodextrins like alpha-, beta- and gamma- cyclodextrin or derivatised cyclodextrins like hydroxypropyl-beta-cyclodextrin (HPBCD; grade: Kleptose↗ HPB) or hydroxypropyl-gamma-cyclodextrin (HPGCD; grade: Cavasol↗ W8 HP Pharma) are formed. The starting experiments are carried out using purified water as a solvent. Solutions of memantine hydrochloride with BCD, HPBCD, and HPGCD with different molar ratios of memantine to cyclodextrin are performed. The liquid and semi solid complexes are investigated by smell and taste masking panels.

The solid complexes, manufactured by granulation, lyophilisaton or spray drying, are investigated by X-ray diffraction (XRD). The XRD experiments are performed on a Stoe instrument. The samples are measured via transmission.

In all cases, complexation occurs spontaneously.

**Table 1 - Additional data (calculation basis)**

| | Molecular weight [g/mol] | Loss on drying [%] | Description, manufacturer |
|---|---|---|---|
| β-Cyclodextrin | 1135 | 11.94% | Kleptose, Roquette |
| γ-Cyclodextrin | 1297 | 7.82% | Cavamax , ISP |
| HBPCD | 1399 | 7.41% | Kleptose, Roquette |
| Memantine-HCl | 215.77 | 0 % | -, Merz Pharmaceuticals |

**Table 2 - Beta-CD experiments**

| Molar ratio Memantine-HCl / β-CD ca. 1:1, 1:2 and 1:3 | | | |
|---|---|---|---|
| | mg / dose | mg / dose | mg / dose |
| Memantine-HCl | 10.0 | 10.0 | 10.0 |
| β-CD | 58.84 | 117.68 | 176.52 |
| Purif. water | Ad 1.0 ml | Ad 1.0 ml | Ad 1.0 ml |

**Table 3 - Gamma-CD experiments**

| Molar ratio Memantine-HCl / γ-CD ca. 1:1, 1:2 and 1:3 | | | |
|---|---|---|---|
| | mg / dose | mg / dose | mg / dose |
| Memantine-HCl | 10.0 | 10.0 | 10.0 |
| γ-CD | 64.76 | 129.52 | 194.28 |
| Purif. water | Ad 1.0 ml | Ad 1.0 ml | Ad 1.0 ml |

**Table 4 - Gamma-CD experiments**

| Molar ratio Memantine-HCl / HPBCD ca. 1:1, 1:2 and 1:3 | | | |
|---|---|---|---|
| | mg / dose | mg / dose | mg / dose |
| Memantine-HCl | 10.0 | 10.0 | 10.0 |
| HPBCD | 69.64 | 139.28 | 208.92 |
| Purif. water | Ad 1.0 ml | Ad 1.0 ml | Ad 1.0 ml |

### Example 2

Semi solid compositions comprising Memantine-HCl and hydroxypropyl-beta-cyclodextrin (HPBCD, grade: Kleptose↗ HPB) are prepared by dissolving weighed amounts of the active compound and the cyclodextrin in measured amounts of purified water. Subsequently, the viscosity controlling excipient is added under stirring. Purified water is then added until the final volume was reached. The solutions are filled into plastic tubes or syringes.

Test solution A exhibits a molar cyclodextrin/memantine ratio of 1 : 1, test solution B a ratio of 3 : 1 and test solution C a ratio of 5:1. The viscosity is controlled via the excipient hypromellose (HPMC) using 1 %, 1.5 % and 2 % to adjust an optimised viscosity.

**Table 5**

| Component | Gel A | Gel B | Gel C |
|---|---|---|---|
| Memantine | 5.0 mg | 10.0 mg | 20.0 mg |
| hydrochloride | | | |
| HPBCD | 32.4 mg | 129.6 mg | 388.8 mg |
| Hypromellose (HPMC) | 10.0 mg | 15.0 mg | 20.0 mg |
| Citrate buffer pH 5.0 | 0.1 ml | 0.1 ml | 0.1 ml |
| Purified water | ad 1.0 ml | ad 1.0 ml | ad 1.0 ml |

In another formulation series several Polyvinylpyrrolidones (PVP) are tested as viscosity controlling agents.

**Table 6**

| Component | Solution A | Solution B | Solution C |
|---|---|---|---|
| Memantine hydrochloride | 5.0 mg | 10.0 mg | 20.0 mg |
| HPGCD | 32.4 mg | 129.6 mg | 388.8 mg |
| PVP | 10.0 mg | 50.0 mg | 100.0 mg |
| Citrate buffer pH 5.0 | 0.1 ml | 0.1 ml | 0.1 ml |
| Purified water | ad 1.0 ml | ad 1.0 ml | ad 1.0 ml |

### Example 3

Equimolar amounts of memantine hydrochloride and either hydroxypropyl-beta-cyclodextrin (HPBCD; grade: Kleptose↗ HPB), hydroxypropyl-gamma-cyclodextrin (HPGCD; grade: Cavasol↗ W8 HP Pharma) or unsubstituted beta-cyclodextrin (BCD; grade: Kleptose↗) are dissolved together in purified water, poured into aluminum cups and subsequently dried by vacuum evaporation in a Rotavapor apparatus. The resulting white samples have a mechanically acceptable stability and have a tablet like form. The samples may be pressed out of the cups without breaking. The solutions and the resulting lyophilysates are analysed by a taste evaluation panel. The equimolar samples of the solution and the lyophilysate are tested by direct ingestion and have a slightly bitter taste. By increasing the amount of the cyclodextrin to the threefold and fivefold amount on a molar ration, the organoleptic properties increase dramatically. Solution B and sample B have a neutral taste and solution C and sample C has a neutral and slightly sweet taste. The solutions and lyophilised samples are without any recogniseable odour.

The disintegration and dissolution behaviour of the samples is improved by milling the samples into granules and powders. The powders dissolve in water within seconds.

**Table 7 - Solution composition prior to lyophilisation**

| Component | Solution A / | Solution B / | Solution C / |
|---|---|---|---|
| | Sample A (1:1) | Sample B (1:3) | Sample C (1:10) |
| Memantine hydrochloride | 20.0 mg | 20.0 mg | 20.0 mg |
| HPBCD | 128.0 mg | 388.8 mg | 1296.0 mg |
| Purified water | ad 1.0 ml | ad 1.0 ml | ad 1.0 ml |

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

All patents, applications, publications, test methods, literature, and other materials cited herein are hereby incorporated by reference.

## Claims

1. A composition comprising a compound selected from those of formula (I) 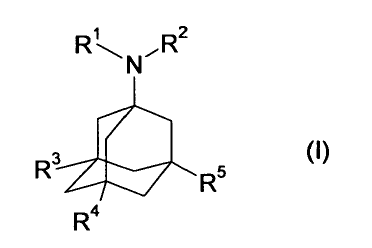 wherein
R¹ and R², which may be the same or different, represent hydrogen or C₁₋₆alkyl, or together represent -(CH₂)ₓ-, wherein x is 2 to 5, inclusive;
R³ and R⁴, which may be the same or different, represent hydrogen, C₁₋₆alkyl, cycloC₅₋₆alkyl, or phenyl; and
R⁵ represents hydrogen or C₁₋₆alkyl, and optical isomers, enantiomers, hydrates, solvates, polymorphs, and pharmaceutically acceptable salts thereof;
and a pharmaceutically acceptable cyclodextrin or combination of pharmaceutically acceptable cyclodextrins.

2. The composition according to Claim 1, wherein the composition is an aqueous liquid composition.

3. The composition according to Claim 1, wherein the composition is a semi-solid composition.

4. The composition according to Claim 1, wherein the composition is a rapidly dissolving solid composition, for reconstitution optionally in form of a powder, granules or a lyophilisate.

5. The composition according to claim 4, wherein reconstitution with an aqueous solvent results in an aqueous liquid composition.

6. The composition according to any preceding Claim, wherein the pharmaceutically acceptable cyclodextrin is selected from alpha-cyclodextrin, beta-cyclodextrin, randomly methylated beta-cyclodextrin, 2-O-methyl-beta-cyclodextrin, heptakis-(2,6-di-O-methyl)-beta-cyclodextrin (dimethyl-beta-cyclodextrin), acetylated dimethyl-beta-cyclodextrin, heptakis-(2,3,6-tri-O-methyl)-beta-cyclodextrin (trimethyl-beta-cyclodextrin, 2-hydroxypropyl-beta-cyclodextrin, sulfoalkylether-beta-cyclodextrin, sulfobutylether-beta-cyclodextrin, O-carboxymethyl-O-ethyl-beta-cyclodextrin, glucuronyl-glucosyl-beta-cyclodextrin, glucosyl-beta-cyclodextrin, maltosyl-beta-cyclodextrin, beta-cyclodextrin sulphate, beta-cyclodextrin phosphate, gamma-cyclodextrin, 2-hydroxypropyl-gamma-cyclodextrin, sulfoalkylether-beta-cyclodextrin, and sulfobutylether-beta-cyclodextrin

7. The composition according to any of Claims 1 to 5, wherein the pharmaceutically acceptable cyclodextrin is selected from optionally hydroxyalkyl-substituted beta- and gamma-cyclodextrins.

8. The composition according to any of Claims 2 to 7, wherein the molar ratio of the cyclodextrin to the compound of formula (I) is at least about 0.1:1

9. The composition according to any preceding claim, wherein the compound of formula (I) is memantine or a pharmaceutically acceptable salt thereof.

10. The composition according to Claim 9, wherein the concentration of memantine or of the pharmaceutically acceptable salt thereof is in the range from about 1 mg/ml to about 100 mg/ml.

11. The composition according to Claim 10, wherein the concentration of memantine or of the pharmaceutically acceptable salt thereof is in the range from about 2 mg/ml to about 75 mg/ml.

12. The composition according to Claim 11, wherein the concentration of memantine or of the pharmaceutically acceptable salt thereof is in the range from about 5 mg/ml to about 50 mg/ml.

13. A medicament comprising the composition according to any preceding claim.

14. The use of a composition according to any of Claims 1 to 12 for the manufacture of a medicament for treating a CNS disorder.

15. A composition according to any of Claims 1 to 12 for the treatment of a CNS disorder or a condition selected from neurodegenerative disorders, Alzheimer's disease, Parkinson's disease, AIDS dementia, neuropathic pain, cerebral ischemia, epilepsy, hepatic encephalopathy, multiple sclerosis, stroke, depression, tardive dyskinesia, autism, ADHD, and autistic spectrum disorders.
